# EUROPEAN PATENT SPECIFICATION

(11) **EP 3 284 815 B1**
(45) Date of publication and mention of the grant of the patent: **28.04.2021**
(21) Application number: 16821684.4
(22) Date of filing: 08.07.2016
(51) Int. Cl.: C12M 1/36, C12M 1/34, C12M 1/26, B25J 9/02, G02B 21/00, C12M 3/00, G01N 35/00, G01N 35/04, C12M 1/00, G02B 21/32, G02B 21/36

(54) **AUTOMATED CELL CULTIVATION DEVICE, AND METHOD FOR OPERATING CULTIVATION DEVICE**
AUTOMATISIERTE ZELLKULTIVIERUNGSVORRICHTUNG UND VERFAHREN ZUM BETRIEB EINER KULTIVIERUNGSVORRICHTUNG
DISPOSITIF DE CULTURE CELLULAIRE AUTOMATISÉ, ET PROCÉDÉ DE FONCTIONNEMENT DE DISPOSITIF DE CULTURE

(30) Priority: 09.07.2015 KR 20150098009; 29.03.2016 KR 20160037323
(43) Date of publication of application: 21.02.2018
(73) Proprietor: Samsung Life Public Welfare Foundation, Seoul 04348 (KR)
(72) Inventor: NAM, Dohyun, Seoul 06334 (KR); RYU, Gyuha, Seoul 06351 (KR); LEE, Jinku, Seoul 06284 (KR)
(74) Representative: Weickmann & Weickmann PartmbB
(86) International application number: PCT/KR2016/007437
(87) International publication number: WO 2017/007278

(56) References cited:
- EP-A1- 1 598 415
- EP-A1- 1 661 980
- WO-A2-2010/121601
- WO-A2-2011/130865
- JP-A- 2002 262 856
- JP-A- 2005 304 303
- JP-A- 2006 055 027
- JP-A- 2007 312 668
- US-A1- 2003 040 104
- US-A1- 2009 029 462

## Description

### [TECHNICAL FIELD]

The present invention relates to an automatic cell culture device and an operating method thereof.

### [BACKGROUND ART]

In general, cell culture is the process by which cells separated from an organism are grown. A primary culture is performed by aseptically selecting a biometric tissue and treating the biometric tissue using a digestive enzyme such as trypsin or pronase to separate single cells from the biometric tissue. In addition, a next subculture is performed by transplanting and inoculating single cells, which is obtained by distributively processing cell lines that are being subcultured using the same enzyme, into a growth medium. A method of distributively growing single cells using proteolytic enzymes refers to the cell culture.

After the 1950s, the cell culture has been started by developing a cell dispersion method by trypsin treatment. Development of the cell culture method enables cells constituting a living thing to be regarded as unicellular organisms. Using a research result acquired in such a method, the basal metabolism, proliferation, differentiation, aging, and tumor virus inspection of a cell may be regarded quantitatively in a cellular level. The cell culture is classified into monolayer culture in which a cell is proliferated while having been attached to a culture device and suspension culture in which a cell is proliferated while having been suspended, instead of having been attached to the culture device. In addition, the cell culture is further classified into single-cell culture in which a single cell is cultured to form a colony and mass culture in which a large number of cells are cultured.

Recently, various researches and developments are being conducted at an accelerated pace in order to precisely and stably perform the cell culture Document EP1598415 A1 discloses an automatic cell culture device comprising: an incubator configured to contain at least one container for culturing cells; a microscope configured to observe a state of the cell in the container; a robot arm configured to move the container; a liquid handler configured to suction liquid from the container; and a control device configured to control an operation of at least one of the incubator, the microscope, the robot arm, and the liquid handler.

### [DETAILED DESCRIPTION OF THE INVENTION]

### [TECHNICAL PROBLEM]

The present invention is intended to provide an automatic cell culture device as defined by claim 1 and an operating method thereof.

### [TECHNICAL SOLUTION

In accordance with claim 1, the present invention provides an automatic cell culture device comprising: an incubator configured to contain at least one container for culturing cells;a microscope configured to observe a state of the cell in the container;a robot arm configured to move the container;a liquid handler comprising a grip part, which is able to move forward or backward along a longitudinal axis road, and to move leftward or rightward along a transversal axis road, wherein the liquid handler is configured to introduce liquid into or discharge liquid from the container and the grip part is configured to hold and move the container;a work part configured to perform at least one of rotating, shaking, and tilting of the container; and a control device configured to control an operation of at least one of the incubator, the microscope, the robot arm, the liquid handler, the grip part and the work part.

Additional aspects will be set forth in part in the description which follows . In accordance with claim 2, the automatic cell culture device may further include a repository configured to store the container to be moved by the robot arm and a centrifugal separator configured to separate a particle of a material included in the container using centrifugal force.

The operating method of the automatic cell culture device according to claim 1 or claim 2 is defined by claim 3.

The operation protocol for the container may be at least one of a protocol for an operation of reinserting the container into the incubator, a protocol for an operation of injecting a culture medium into the container, and a protocol for an operation of performing sub-culturing using the container.

When the protocol for an operation of injecting the cultural medium into the container is selected, the driving of the robot arm may include enabling the robot arm to move the container extracted from the incubator or the repository to a loader, and the operating method may further include moving the container moved to the loader to a first work part using a grip part included in the liquid handler; suctioning a culture medium provided in a culture medium storage using the liquid handler and dividing the suctioned culture medium into the container positioned in the first work part; moving the container into which the culture medium is divided to the loader using the grip part; and reinserting the container moved to the loader into the incubator.

When the protocol for an operation of performing sub-culturing using the container is selected, the driving of the robot arm may include enabling the robot arm to move the container extracted from the incubator or the repository to a loader, and the operating method may further includes moving the container moved to the loader to the first work part using the grip part included in the liquid handler, transferring a material included in the container moved to the first work part to a container having a predetermined shape positioned in a second work part, moving the container having a predetermined shape to the centrifugal separator to perform centrifugation on the container, processing a particle separated through the centrifugation, observing the processed particle using the microscope; transferring the processed particle to a new container; and reinserting the new container into the incubator.

The container having a predetermined shape may be a tubular container having a stopper, for example, an eppendorf tube (E-tube) or a centrifuge tube (C-tube). The transferring of a material included in the container moved to the first work part to a container having a predetermined shape positioned in the second work part may further include transferring a mixed liquid acquired by dividing a predetermined solution into the container moved to the first work part and shaking the container to the container having a predetermined shape.

The processing of a particle separated through the centrifugation may include removing a supernatant liquid generated in the container through the centrifugation, injecting a predetermined material into the container having a predetermined shape to perform mixing and suspension, performing centrifugation on the container that contains the suspended material, removing the supernatant liquid generated in the container through the centrifugation, injecting the culture medium into the container to perform mixing, and mixing the predetermined material and a cell suspension using another container having a predetermined shape.

The predetermined material may be an enzyme or phosphate-buffered saline (PBS).

The observing of the processed particle using the microscope may further include transferring the material included in the container having a predetermined shape to an auxiliary observation device before observing the processed particle through the microscope. The auxiliary observation device includes a microscopic chip (e.g., C-chip) for measuring a cell count.

The transferring of the processed particle to a new container may further include dividing the culture medium into the new container before the transfer of the particle, and the material including the processed particle may be transferred to the new container into which the culture medium is divided.

A non-transitory computer-readable recording medium having recorded thereon a program for executing the method of any one of claims 3-11 is provided.

### [ADVANTAGEOUS EFFECTS OF THE INVENTION]

As described above, according to the one or more of the above exemplary embodiments, it is possible to systematically culture a cell by applying a certain culture protocol using the automatic cell culture device, thereby minimizing a deviation between experimenters.

It is also possible to safely culture a cell by minimizing experimental infection or tumor cell contact that may occur when a tumor cell is cultured.

In addition, by using the automatic cell culture device according to an embodiment of the present disclosure, it is possible to stably and precisely culture a cell, separate a cell (e.g., through the centrifugation, etc.), or process a cell (e.g., add an enzyme, perform pipetting, add a culture medium, etc.).

Furthermore, by using the automatic cell culture device according to an embodiment of the present disclosure, it is possible to quickly and efficiently dividing a trypan blue solution, which is used to check the ratio of dead cells to live cells, into cells and check the cells (e.g., counting, etc.).

### [DESCRIPTION OF THE DRAWINGS]

FIG. 1A is a block diagram of an automatic cell culture device according to an embodiment of the present disclosure;
FIG. 1B is a block diagram of an automatic cell culture device according to another embodiment of the present disclosure;
FIG. 1C is a cross-sectional view of a centrifugal separator according to an embodiment of the present disclosure;
FIG. 2 shows an example of an automatic cell culture device according to an embodiment of the present disclosure;
FIG. 3 is a plan view showing a work space of an automatic cell culture device according to an embodiment of the present disclosure;
FIG. 4 is a flowchart showing an operating method of an automatic cell culture device according to an embodiment of the present disclosure;
FIG. 5 is a flowchart showing an operating method of an automatic cell culture device based on a selected protocol according to an embodiment of the present disclosure;
FIGS. 6A to 6C are flowcharts showing an operating method of an automatic cell culture device according to a selected protocol;
FIG. 7 is a plan view of an automatic cell culture device from which a robot arm has been removed which is not part of the present invention;
FIG. 8 shows an example of a cooler according to an embodiment of the present disclosure;
FIG. 9 shows an example of a heater according to an embodiment of the present disclosure;
FIG. 10 shows an example of a work part according to an embodiment of the present disclosure;
FIG. 11 shows an example of a grip part according to an embodiment of the present disclosure;
FIG. 12 shows an example of a decapper according to an embodiment of the present disclosure;
FIG. 13 shows an example of a microscope according to an embodiment of the present disclosure;
FIG. 14 shows an example of an incubator and an incubator loader according to an embodiment of the present disclosure; and
FIG. 15A shows an example of an image acquisition unit and FIG.15B shows an example of an acquired image screen according to an embodiment of the present disclosure.

### [BEST MODE]

The automatic cell culture device as defined by claim 1 may further include a repository configured to store the container to be moved by the robot arm and a centrifugal separator configured to separate a particle of a material included in the container using centrifugal force.

The operating method of the automatic cell culture device according claim 1 or claim 2 is defined by claim 3.

The operation protocol for the container may be at least one of a protocol for an operation of reinserting the container into the incubator, a protocol for an operation of injecting a culture medium into the container, and a protocol for an operation of performing sub-culturing using the container.

When the protocol for an operation of injecting the cultural medium into the container is selected, the driving of the robot arm may include enabling the robot arm to move the container extracted from the incubator or the repository to a loader, and the operating method may further include moving the container moved to the loader to a first work part using a grip part included in the liquid handler; suctioning a culture medium provided in a culture medium storage using the liquid handler and dividing the suctioned culture medium into the container positioned in the first work part; moving the container into which the culture medium is divided to the loader using the grip part; and reinserting the container moved to the loader into the incubator.

When the protocol for an operation of performing sub-culturing using the container is selected, the driving of the robot arm may include enabling the robot arm to move the container extracted from the incubator or the repository to a loader, and the operating method may further includes moving the container moved to the loader to the first work part using the grip part included in the liquid handler, transferring a material included in the container moved to the first work part to a container having a predetermined shape positioned in a second work part, moving the container having a predetermined shape to the centrifugal separator to perform centrifugation on the container, processing a particle separated through the centrifugation, observing the processed particle using the microscope; transferring the processed particle to a new container; and reinserting the new container into the incubator.

The container having a predetermined shape may be a tubular container having a stopper, for example, an eppendorf tube (E-tube) or a centrifuge tube (C-tube). The transferring of a material included in the container moved to the first work part to a container having a predetermined shape positioned in the second work part may further include transferring a mixed liquid acquired by dividing a predetermined solution into the container moved to the first work part and shaking the container to the container having a predetermined shape.

The processing of a particle separated through the centrifugation may include removing a supernatant liquid generated in the container through the centrifugation, injecting a predetermined material into the container having a predetermined shape to perform mixing and suspension, performing centrifugation on the container that contains the suspended material, removing the supernatant liquid generated in the container through the centrifugation, injecting the culture medium into the container to perform mixing, and mixing the predetermined material and a cell suspension using another container having a predetermined shape.

The predetermined material may be an enzyme or phosphate-buffered saline (PBS).

The observing of the processed particle using the microscope may further include transferring the material included in the container having a predetermined shape to an auxiliary observation device before observing the processed particle through the microscope. The auxiliary observation device includes a microscopic chip (e.g., C-chip) for measuring a cell count.

The transferring of the processed particle to a new container may further include dividing the culture medium into the new container before the transfer of the particle, and the material including the processed particle may be transferred to the new container into which the culture medium is divided.

According to one or more exemplary embodiments, a non-transitory computer-readable recording medium having recorded thereon a program for executing the method is provided.

### [MODE OF THE INVENTION]

Hereinafter, embodiments of the present disclosure will be described in detail to be easily embodied by those of ordinary skill in the art with reference to the accompanying drawings. The exemplary embodiments may, however, be embodied in many different forms and should not be construed as being limited to the embodiments set forth herein. In the accompanying drawings, portions irrelevant to a description of the exemplary embodiments will be omitted for clarity. Moreover, like reference numerals refer to like elements throughout.

As used herein, the term "and/or" includes any and all combinations of one or more of the associated listed items. Expressions such as "at least one of," when preceding a list of elements, modify the entire list of elements and do not modify the individual elements of the list.

Terms used herein will be briefly described, and the exemplary embodiments will be described in detail below.

As the terms used herein, so far as possible, widely-used general terms are selected in consideration of functions in the present disclosure; however, these terms may vary depending on the intentions of those skilled in the art, the precedents, or the appearance of new technology. Also, if there is a term which is arbitrarily selected by the applicant in a specific case, in this case, a meaning of the term will be described in detail in a corresponding description portion of the exemplary embodiments. Therefore, the terms should be defined on the basis of the entire content of this specification instead of a simple name of each of the terms.

Furthermore, when one part is referred to as "comprising (or including or having)" other elements, it should be understood that it can comprise (or include or have) only those elements, or other elements as well. Moreover, each of terms such as "unit" and "module" described in the specification denotes an element for performing at least one function or operation, and may be implemented in hardware, software or the combination of hardware and software. In this disclosure below, when one part (or element, device, etc.) is referred to as being "connected" to another part (or element, device, etc.), it should be understood that the former can be "directly connected" to the latter, or "electrically connected" to the latter via an intervening part (or element, device, etc.).

Hereinafter, embodiments of the present disclosure will be described in detail with reference to the accompanying drawings.

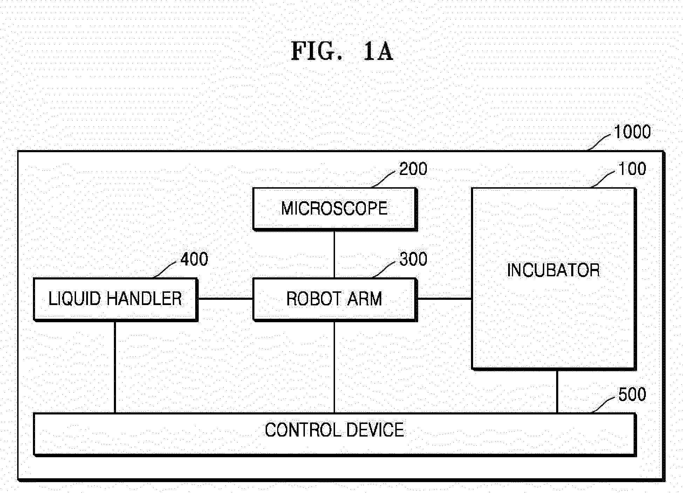

FIG. 1A is a block diagram of an automatic cell culture device according to an embodiment of the present disclosure.

An automatic cell culture device 1000 according to an embodiment of the present disclosure is defined by claim 1.

For example, there may be at least one container that contains a cell in the incubator 100 of the automatic cell culture device 1000. In other words, a cell may be cultured in the incubator 100 for a predetermined time. For example, the predetermined time may be one day or one week, and a time for which the cell should be present in the incubator 100 according culture conditions of the cell may be extended or shortened to two or three days. The container that contains a cell may be, for example, a 6-well plate, but is not limited thereto. The term "container" used herein may refer to a container that may contain at least one of a cell and a cell suspension.

According to an embodiment of the present disclosure, the microscope 200 may be an inverted microscope, and a lens may be automatically replaced with a new one. For example, the lens may be replaced with a lens having magnitude of x1.25, x4, x10, or x20 on the basis of a lens replacement signal applied from the control device 500. The color, area, or size of a culture medium may be precisely and accurately observed using the microscope 200. In addition, a culture state of the cell may also be observed.

The robot arm 300 according to an embodiment of the present disclosure may include a load unit disposed at one end and configured to grip various kinds of containers, and may be implemented in a multi-joint form that may be manipulated by at least one motor. Accordingly, the robot arm 300 may move forward, backward, left, and right or rotate while maintaining a horizontal or vertical state of the load unit. The above-described operation of the robot arm 300 may be controlled by the control device 500. In addition, the robot arm 300 may be disposed at the center of the automatic cell culture device 1000 and used to move various kinds of containers or may be additionally disposed at one side and utilized to process a cell.

The liquid handler 400 according to an embodiment of the present disclosure may transfer a container or a solution. The liquid may be introduced into or discharged from the container using a pipette that may be included in the liquid handler 400. In addition, the container may be held and moved using a grip part included in the liquid handler 400.

The control device 500 according to an embodiment of the present disclosure may be a personal computer, a laptop, a tablet PC, a smartphone, a PDA, or a wearable device such as a smart watch. In other words, the control device 500 may denote a device that may transmit or receive data to or from another device in a wired or wireless manner. In addition, the control device 500 may include a display function and may provide a user with operation state information, cell information, etc. of the automatic cell culture device 1000 in real time.

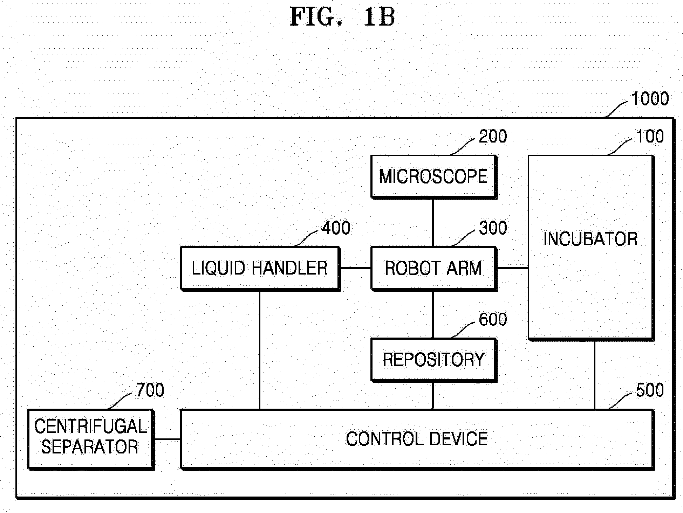

FIG. 1B is a block diagram of an automatic cell culture device according to another embodiment of the present disclosure.

In addition, the automatic cell culture device 1000 according to another embodiment may further include a repository 600 configured to store a container to be moved by the robot arm 300 and a centrifugal separator 700 configured to separate a particle of a material included in the container using centrifugal force.

The repository 600 according to an embodiment of the present disclosure may include at least one stand bracket that may temporarily or shortly store various kinds of containers. In addition, the repository 600 may rotate on the basis of a signal applied from the control device 500. As described above, the robot arm 300 may transfer the various kinds of containers to the repository 600 and also extract the various kinds of containers from the repository 600 and reinsert the extracted containers into the incubator 100. That is, the robot arm 300 may freely change positions of the various kinds of containers.

The centrifugal separator 700 according to an embodiment of the present disclosure is configured to separate a cell containd in the container (e.g., a C-tube), that is, separate a cell layer using centrifugal force.

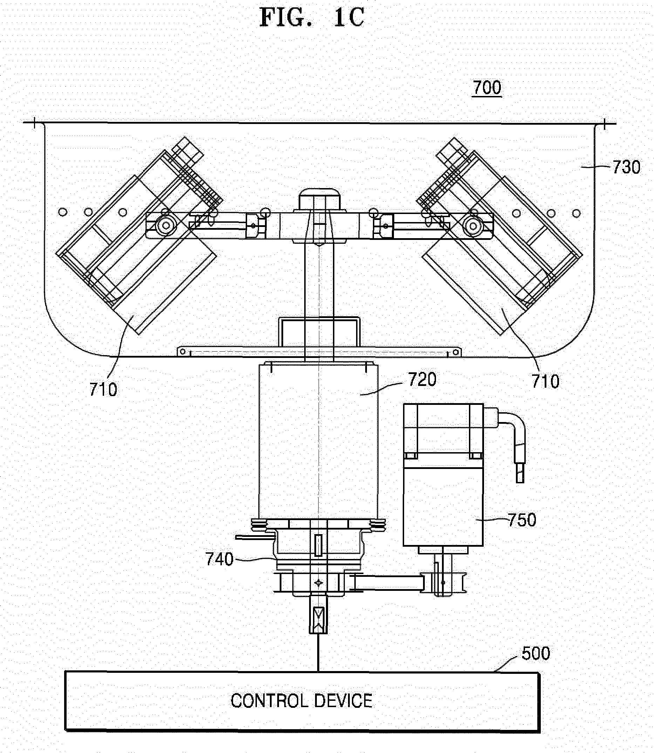

FIG. 1C is a cross-sectional view of a centrifugal separator according to an embodiment of the present disclosure.

The centrifugal separator 700 according to an embodiment of the present disclosure may include a bucket 710 configured to contain a container, a high speed motor 720 configured to rotate the bucket 710 at high speed, a high speed rotator 730 configured to receive rotational force from the high speed motor 720 and rotate the container, the high speed rotator 730 being a high speed rotation space in which at least one loads are included, an electromagnet clutch 740 configured to sense a rotational speed of the high speed motor 720 and stop the rotation of the bucket 710 using an electromagnet when the rotational speed sufficiently decreases (e.g., 1 r/min, etc.), and a position control motor 750 configured to rotate the high speed motor 720 and the electromagnet clutch 740 at low speed in an original rotation direction or an opposite direction to move the bucket 710 to a destination position of a user. The destination position of the user may be a position (e.g., a start position) at a time when the container is inserted into the bucket 710 before the centrifugation. The centrifugal separator 700 according to an embodiment of the present disclosure may measure the amount of solution to be centrifuged in the container using a camera and then automatically fill a container contained in a bucket, which is positioned opposite to the bucket accommodating the container that contains the solution to be centrifuged, with the same amount of water. Thus, a principle may be applied to stably maintain the center of gravity, which may be required upon the centrifugation.

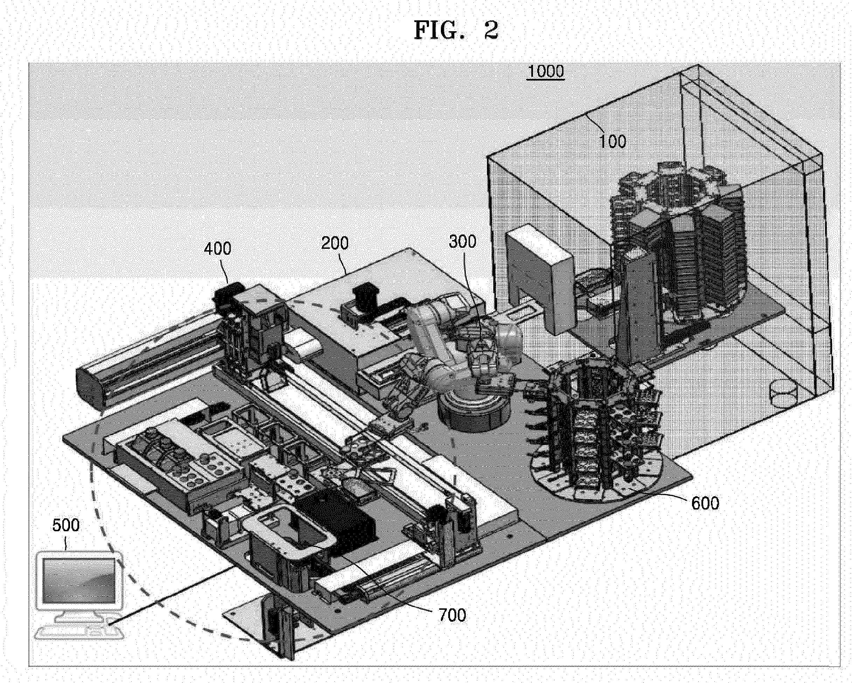

FIG. 2 shows an example of an automatic cell culture device according to an embodiment of the present disclosure.

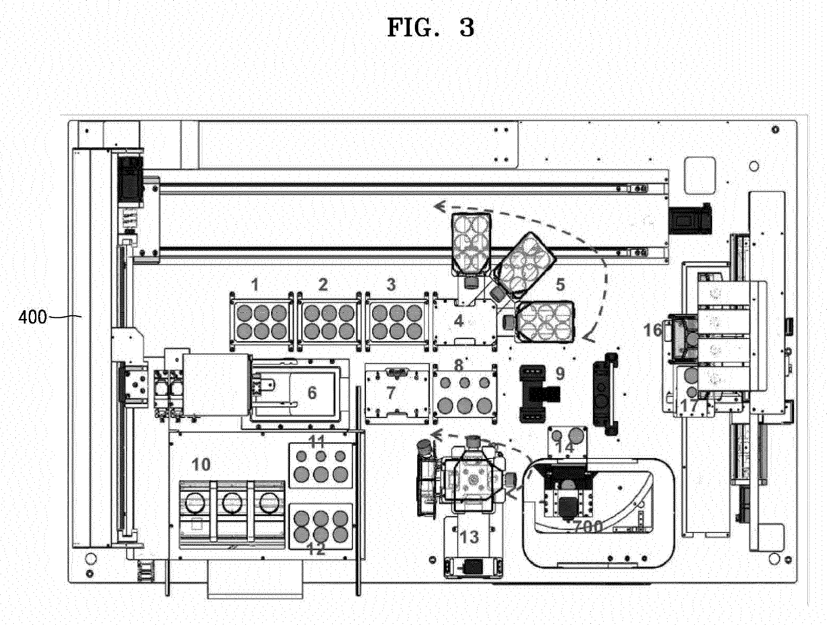

FIG. 2 is merely a schematic diagram for description, and the automatic cell culture device 1000 according to an embodiment of the present disclosure may be implemented in various structures and forms. In addition, the automatic cell culture device 1000 may further include a repository 600 and a centrifugal separator 700. The automatic cell culture device 1000 has a work space (e.g., a dotted circle area in FIG. 2) for observing, measuring, or processing a cell, such as a first work part, a second work part, etc. This will be described below with reference to FIG. 3.

FIG. 3 is a plan view showing a work space of an automatic cell culture device according to an embodiment of the present disclosure.

A process of observing, measuring, or processing a cell may be performed in a work space of an automatic cell culture device 1000 according to an embodiment of the present disclosure. As shown in FIG. 3, at least one pipette storage 1 or 2, a C-tube storage 3, a buffer zone 4 or 14, and a loader 5 may be included in the work space. The loader 5 may move in a predetermined direction and at a predetermined angle. In addition, the automatic cell culture device 1000 may further include a waste storage 6, a first work part 7, a second work part 8, an image acquisition unit 9, and a culture medium container 10. For example, the image acquisition unit 9 may include at least one camera and at least one illuminator. The camera may acquire an image of a container including a cell and may determine a cell layer or the like on the basis of a brightness level, a saturation level, a pixel value, etc. In other words, the camera may apply a general image processing technique to the acquired image to determine a boundary of the image, and may relatively accurately estimate the cell layer or the like according to the determined boundary.

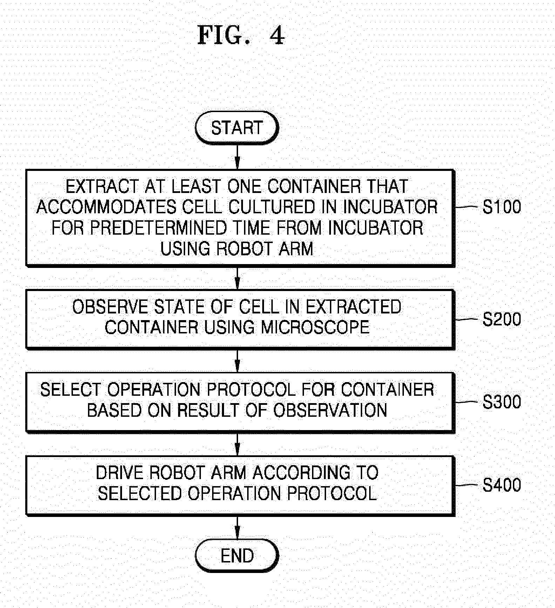

In addition, the automatic cell culture device 1000 may further include a first culture medium (material) storage 11, a second culture medium (material) storage 12, a T-flask work part 13, a decapper 16 or 17, a liquid handler 400, and a centrifugal separator 700. The T-flask work part 13 may more up or down a T-flask once or more times on the basis of a signal applied from the control device 500. In addition, the decapper may be a T-flask decapper 16 or a C-tuber decapper 17. The liquid handler 400 may move forward or backward along a longitudinal axis road. In addition, the liquid handler 400 may move left or right along a transverse axis road. An operation of the automatic cell culture device 1000 according to an embodiment of the present disclosure will be described below with reference to FIGS. 4 to 6C.

FIG. 4 is a flowchart showing an operating method of an automatic cell culture device according to an embodiment of the present disclosure.

An operating method of an automatic cell culture device 1000 according to an embodiment of the present disclosure may include extracting at least one container that contains a cell cultured in an incubator 100 for a predetermined time from the incubator 100 using a robot arm 300 (S100), observing a state of the cell in the extracted container using a microscope 200 (S200), selecting an operation protocol for the container on the basis of a result of the observation (S300), and driving the robot arm 300 according to the selected operation protocol (S400).

The cell may be cultured in the incubator 100 of the automatic cell culture device 1000, for example, for three or four days according to an embodiment of the present disclosure. The container that contains the cultured cell may be moved from the incubator 100 to a work space in order to observe, measure, or process the cultured cell. The container that contains the cell cultured in the incubator 100 for a predetermined time (e.g., three to four days) may be extracted from the incubator 100 by the robot arm 300 (S100). A state of the cell in the extracted container may be observed using the microscope 200 (S200). In other words, the state of the cell may be photographed through the microscope 200. The microscopic image of the cell acquired through the photographing may be transferred to the control device 500 and then displayed. A user may observe or analyze the microscopic image of the cell to select an operation protocol for the container that contains the cell (S300). In other words, an operation of the automatic cell culture device 1000 may be controlled by the control device 500 according to the operation protocol selected by the user (S400). According to the selected operation protocol, the robot arm 300 is driven to change the position of the container. While the position is being changed, various works (e.g., observation, measurement, or processing of the cultured cell) may be performed on the container.

The operation protocol for the container according to an embodiment of the present disclosure may be at least one of a protocol for an operation of reinserting the container into the incubator 100, a protocol for an operation of injecting a culture medium into the container, and a protocol for an operation of performing sub-culturing using the container.

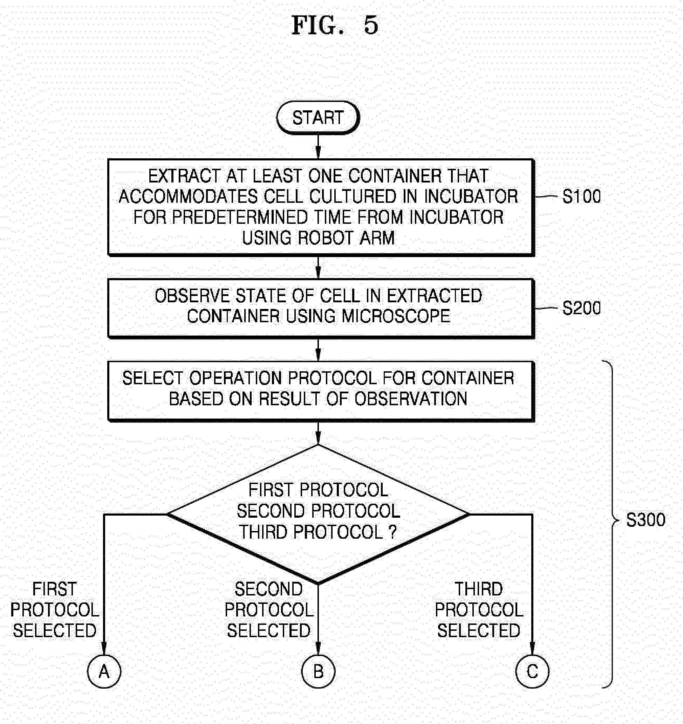

FIG. 5 is a flowchart showing an operating method of an automatic cell culture device based on a selected protocol according to an embodiment of the present disclosure.

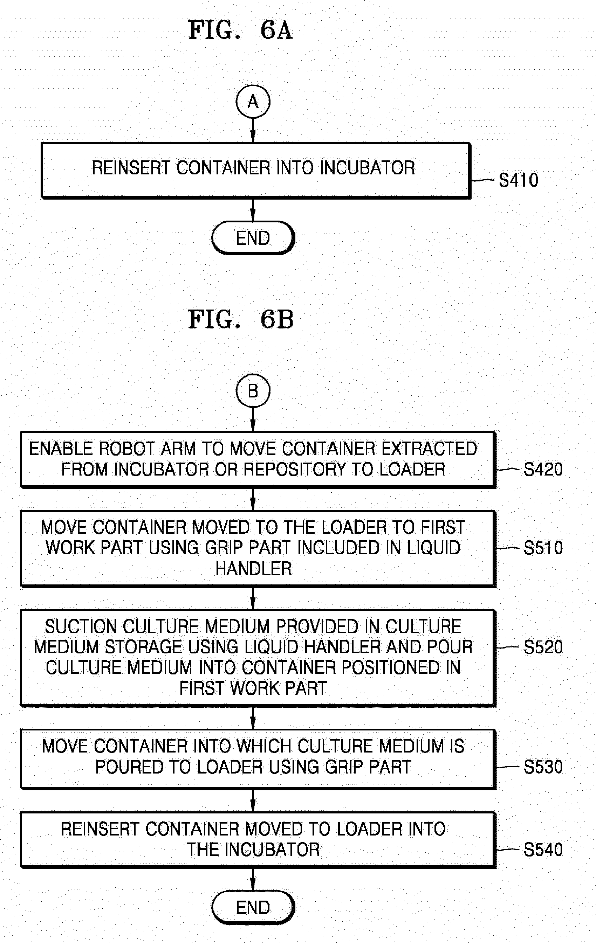

As described above with reference to FIG. 4, an operating method of an automatic cell culture device 1000 according to an embodiment of the present disclosure may include extracting at least one container that contains a cell cultured in an incubator 100 for a predetermined time from the incubator 100 using a robot arm 300 (S100), observing a state of the cell in the extracted container using a microscope 200 (S200), selecting an operation protocol for the container on the basis of a result of the observation (S300), and driving the robot arm 300 according to the selected operation protocol (S400). In other words, when the first protocol is selected as shown in FIG. 5, the method may proceed to step A in association with the driving of the robot arm 300. In addition, the method may proceed to step B in association with the driving of the robot arm 300 when the second protocol is selected, and may proceed to step C in association with the driving of the robot arm 300 when the third protocol is selected. For example, the first protocol may be a protocol for an operation of reinserting the container into the incubator 100. In addition, the second protocol may be a protocol for an operation of injecting a culture medium into the container, and the third protocol may be a protocol for an operation of performing sub-culturing using the container. The above-described proceeding to steps A, B, and C will be described below with reference to FIGS. 6A to 6C.

FIGS. 6A to 6C are flowcharts showing an operating method of an automatic cell culture device according to a selected protocol.

As shown in FIG. 6A, when the first protocol, which may be the protocol for the operation of reinserting the container into the incubator 100, is selected according to an embodiment of the present disclosure, the container may be reinserted into the incubator 100 by the robot arm 300 (S410). In other words, according to the selected operation protocol (e.g., the first protocol), the driving of the robot arm 300 (S400) may include reinserting the container into the incubator 100 (S410).

As shown in FIG. 6B, when the second protocol, which may be the protocol for the operation of injecting the culture medium into the container, is selected according to an embodiment of the present disclosure, the driving of the robot arm 300 (S400) may include enabling the robot arm 300 to move the container extracted from the incubator 100 or the repository 600 to a loader 5 (S420). In addition, the operating method of the automatic cell culture device 1000 may further include moving the container moved to the loader 5, to a first work part 7 using a grip part included in the liquid handler 400 (S510), suctioning a culture medium provided in a culture medium storage using the liquid handler 400 and dividing the culture medium into the container positioned in the first work part 7 (S520), moving the container into which the culture medium is divided to the loader 5 using the grip part (S530), and reinserting the container moved to the loader 5 into the incubator 100 (S540).

The robot arm 300 may move the container extracted from the incubator 100 or the repository 600 to the loader 5 on the basis of a signal applied from the control device 500. As described above, the container may be a cell container such as a 6-well plate. The container moved to the loader 5 may be moved to the first work part 7 by the grip part included in the liquid handler 400. When the 6-well plate moved to the loader 5 has a cap, the cap of the 6-well plate may be opened. The culture medium provided in a second culture medium (material) storage 12 may be suctioned using the liquid handler 400 and then be divided into the container positioned in the first work part 7. After the culture medium is divided, the cap of the 6-well plate may be closed. The container into which the culture medium is divided may be moved to the loader 5 by the grip part. The container moved to the loader 5 may be reinserted into the incubator 100.

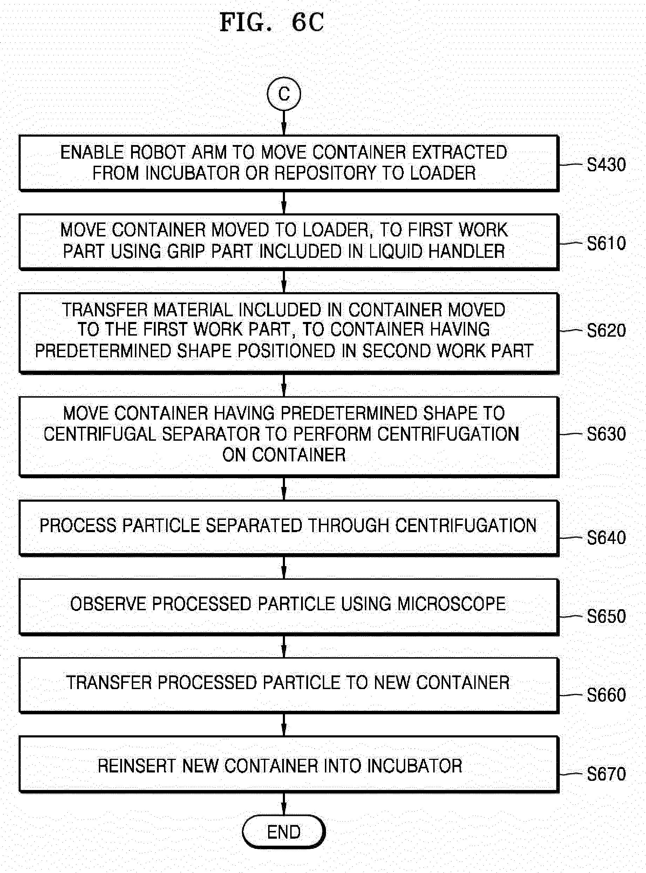

In addition, as shown in FIG. 6C, when the third protocol, which may be the protocol for the sub-culturing operation using the container, is selected according to an embodiment of the present disclosure, the driving of the robot arm 300 (S400) may include enabling the robot arm 300 to move the container extracted from the incubator 100 or the repository 600 to the loader 5 (S430). In addition, the operating method of the automatic cell culture device 1000 may further include moving the container moved to the loader 5, to the first work part 7 using the grip part included in the liquid handler 400 (S610), transferring a material included in the container moved to the first work part 7, to a container having a predetermined shape positioned in a second work part 8 (S620), moving the container having a predetermined shape to the centrifugal separator 700 to perform centrifugation on the container having a predetermined shape (S630), processing a particle separated through the centrifugation (S640), observing the processed particle using the microscope 200 (S650), transferring the processed particle to a new container (S660), and reinserting the new container into the incubator 100.

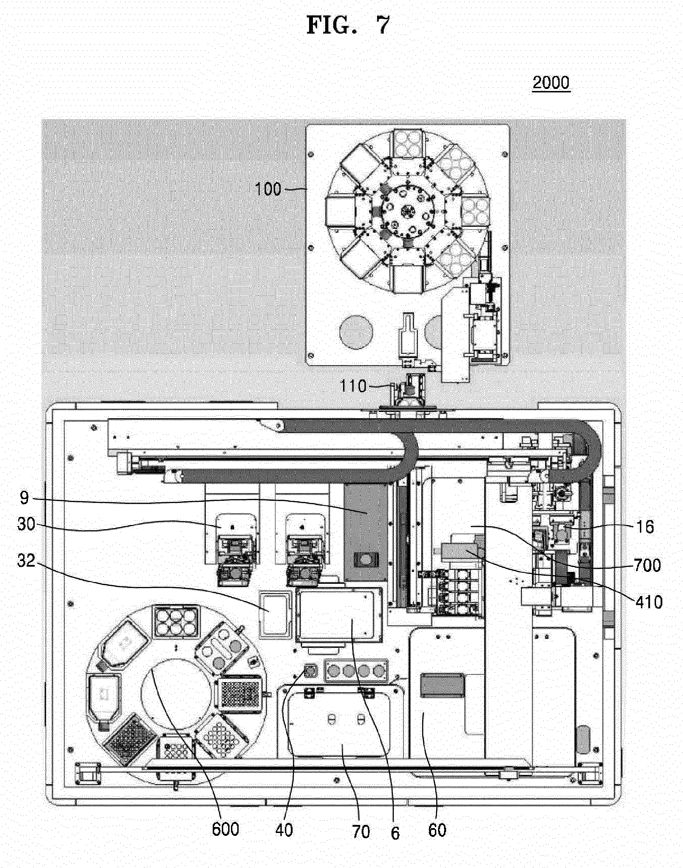

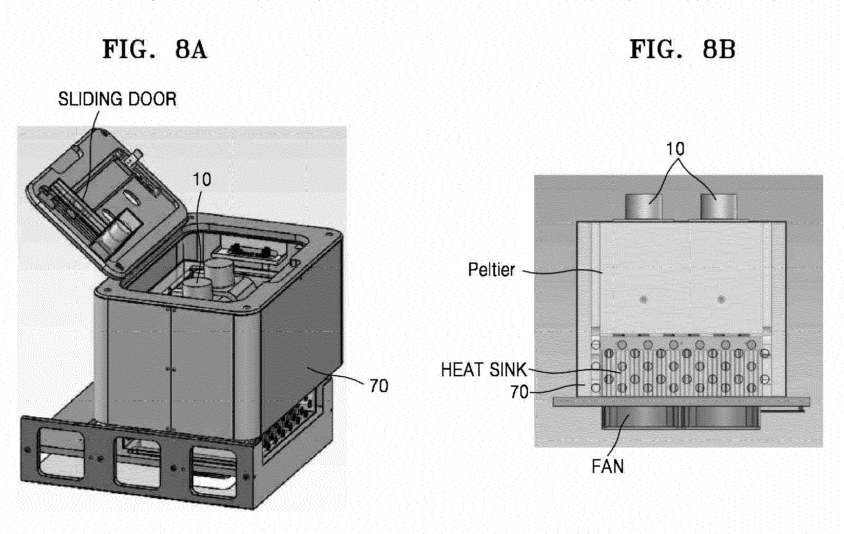

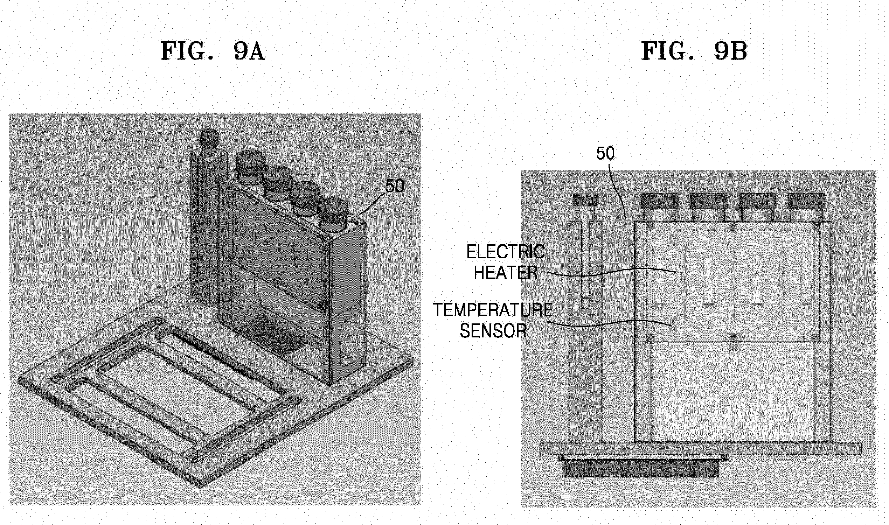

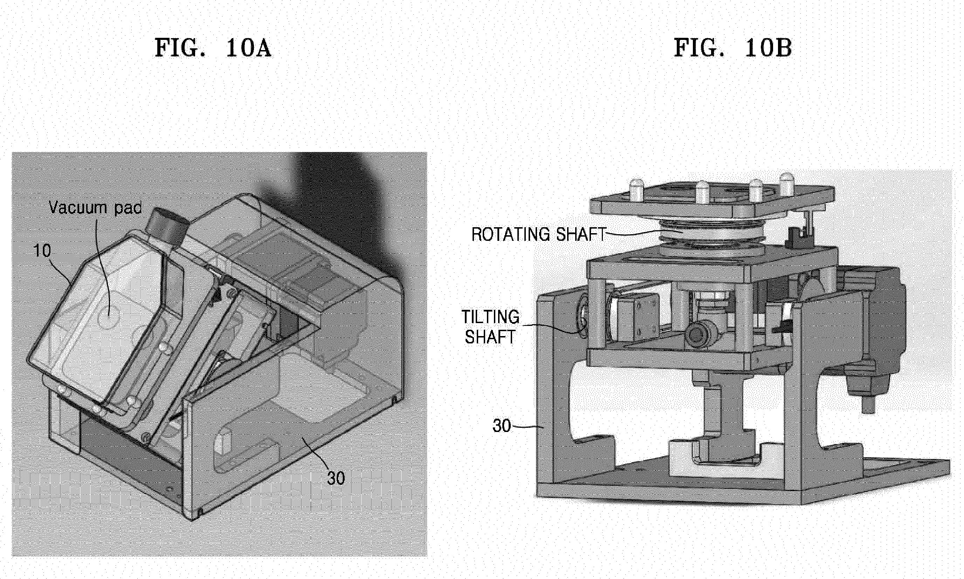

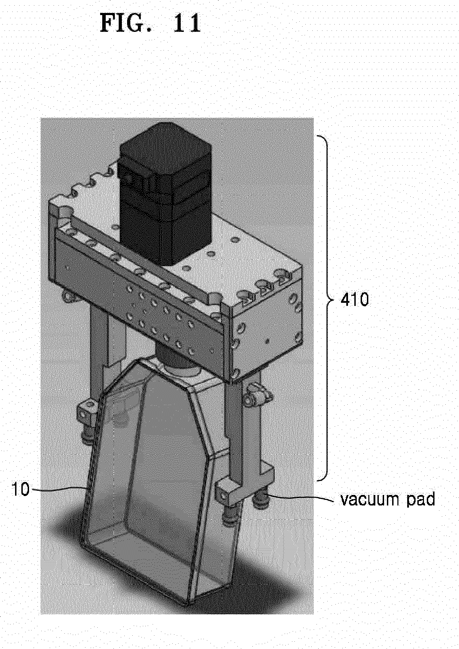

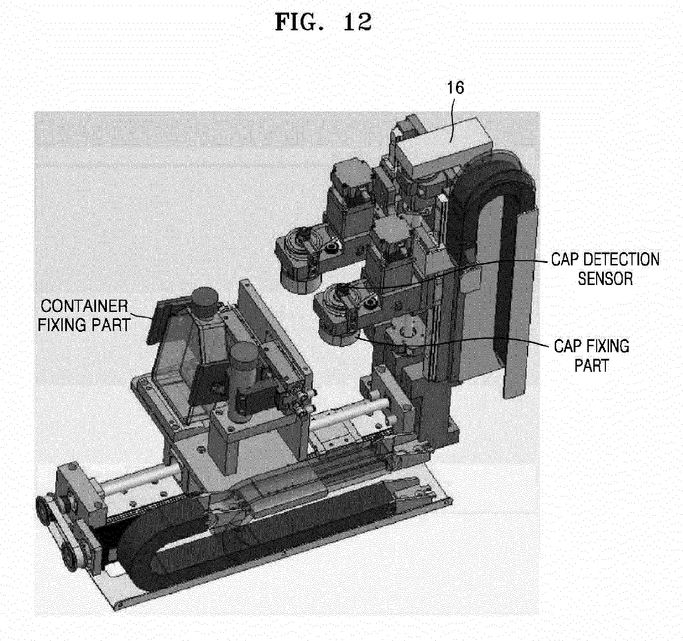

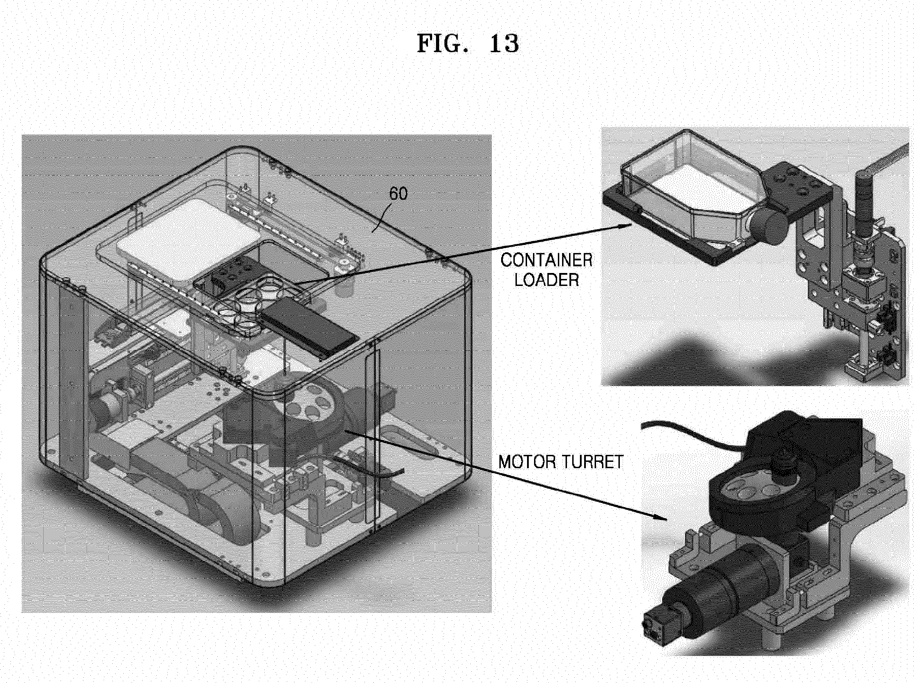

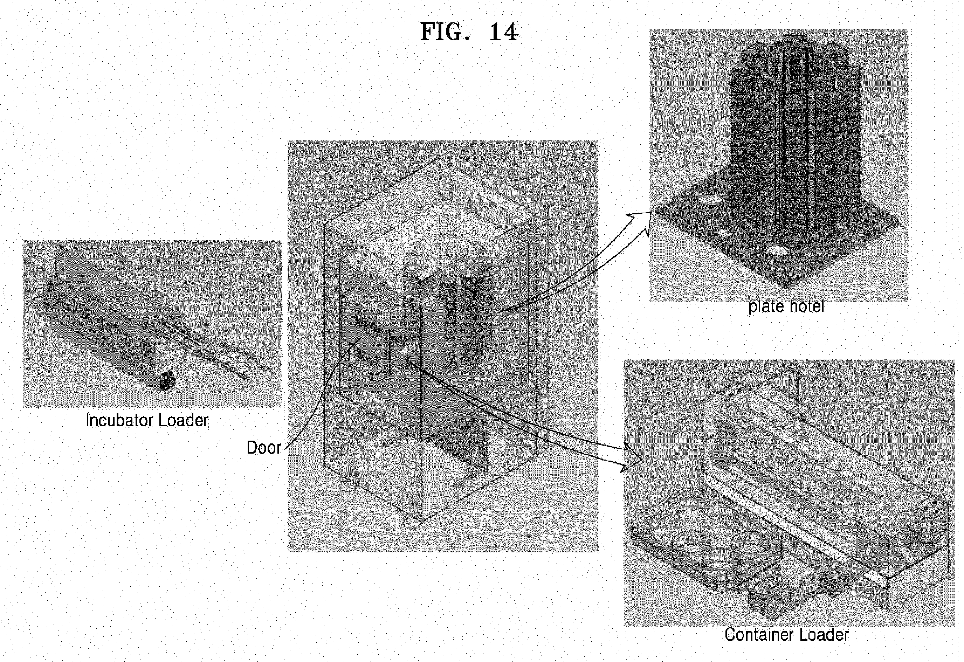

FIG. 7 is a plan view of an automatic cell culture device from which a robot arm has been removed which is not part of the present invention. FIG. 8 shows an example of a cooler according to an embodiment of the present disclosure. FIG. 9 shows an example of a heater according to an embodiment of the present disclosure. In addition, FIG. 10 shows an example of a work part according to an embodiment of the present disclosure. FIG. 11 shows an example of a grip part according to an embodiment of the present disclosure. FIG. 12 shows an example of a decapper according to an embodiment of the present disclosure. FIG. 13 shows an example of a microscope according to an embodiment of the present disclosure. FIG. 14 shows an example of an incubator and a loader of the incubator according to an embodiment of the present disclosure. For reference, FIGS. 8 to 10A and 10B are views having different viewpoints.

Referring to FIG. 7, the robot arm 300 may have been removed from an automatic cell culture device 2000 which is thus not part of the present invention. Since the robot arm 300 has been removed, the automatic cell culture device 2000 may be further reduced in size, compared to the above-described automatic cell culture device 1000. However, a grip part 410 may replace a role of the robot arm 300 that is removable. Accordingly, although the robot arm 300 is removed, a function similar to that of the automatic cell culture device 1000 may be reliably performed by the grip part 410, etc.

As shown in FIG. 7, the automatic cell culture device 2000 includes a waste storage 6, an image acquisition unit 9, a decapper 16, a 6-well container cover deck 32, a work part 30, an enzyme tube deck 40, a heater 50, a microscope 60, a cooler 70, an incubator 100, an incubator loader 110, a grip part 410, a repository 600, a centrifugal separator 700, etc. The automatic cell culture device 2000 may be connected with a control device 500 by wire or wirelessly.

Referring to FIG. 8, the cooler 70 according to an embodiment of the present disclosure includes a Peltier device, a heat sink, and a cooling fan. The temperature in the cooler 70 may be maintained at a certain level through a sliding door. In other words, the cooler 70 may provide a cooling function for maintaining the temperature of a culture medium or PBS solution in the container 10 at approximately 4°C. The temperature may be adjusted, for example, by supplying an electric current to the Peltier device. In addition, referring to FIG. 9, the culture medium or PBS solution cooled by the cooler 70 may also be heated by the heater 50 including an electric heater and a temperature sensor. For example, the culture medium or PBS solution may be heated up to approximately 37°C by the heater 50.

Referring to FIG. 10, at least one of rotating, shaking, and tilting of the transferred container 10 may be performed in the work part 30. In other words, various movements may be performed on the 6-well container, 75 T-Flask, etc. In order to reliably fix the container 10, at least one vacuum pad may be mounted on a portion in which the work part 30 and the container 10 are in contact with each other. As shown in FIG. 10, there may be a rotating shaft for rotating the container 10 at the top of the work part 30, and there may also be a tilting shaft for tilting the container 10 at the middle thereof.

Referring to FIG. 11, the grip part 410 may be a motor grip part that may grip and transfer the container 10 such as 6-well plate, T-Flask, C-tube, etc. In addition, the grip part 410 may be a vacuum grip part equipped with a vacuum pad at one end, as shown in FIG. 11. The vacuum grip part may transfer a cap of the 6-well plate using the vacuum pad, etc.

Referring to FIG. 12, the decapper 16 may function to open or close caps of the C-Tube and the T-Flask a cover. For example, after the container 10 is fixed to a container fixing part, the decapper 16 moves a position for opening the cap. After a cap fixing part moves down, the decapper 16 rotates in a direction in which the cap is opened in order to open the cap of the container 10. In addition, similarly, after the container 10 is fixed to the container fixing part, the decapper 16 moves a position for closing the cap. After the cap fixing part moves down, the decapper 16 rotates in a direction in which the cap is closed in order to close the cap of the container 10. A state in which the cap is opened or closed may be checked in real time using a cap detection sensor included in the decapper 16.

As shown in FIG. 13, the automatic cell culture device 2000 includes the microscope 60 for observing a cell culture state. The microscope 60 may acquire images while moving the container up, down, left, or right using a container loader provided inside the microscope 60. In addition, the microscope 60 may change a lens magnification of the microscope 60 using a motor turret.

Referring to FIG. 14, the automatic cell culture device 2000 includes an incubator 100 and an incubator loader 110 for carrying the container 10 to or out of the incubator 100. The incubator loader 110 may be used to carry the container 10 that may be introduced or discharged through a door of the incubator 100. In other words, when the door of the incubator 100 is opened, and the container 10 positioned over the incubator loader 110 enters the incubator 100, the container loader may receive the container 100 and then position the container 10 in a plate hotel. The plate hotel may be rotated using a motor mounted at the center of the bottom.

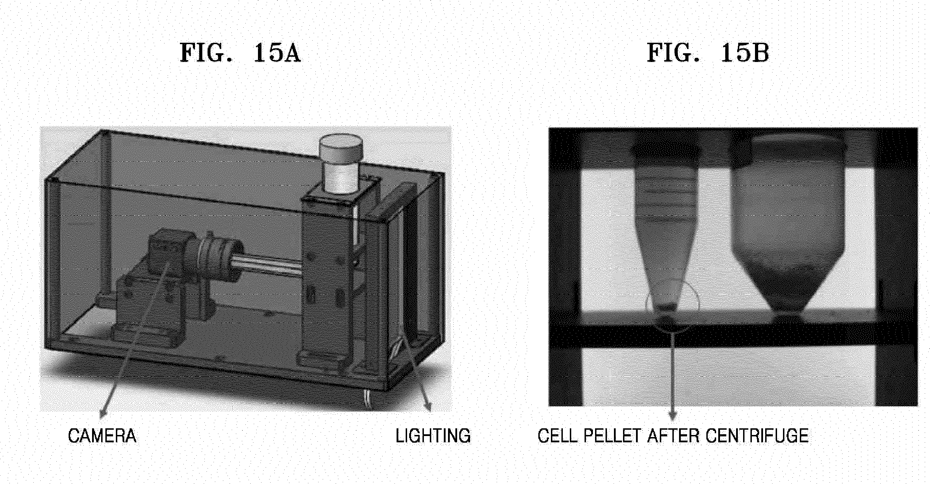

FIG. 15 shows (a) an example of an image acquisition unit and (b) an example of an acquired image screen according to an embodiment of the present disclosure.

According to an embodiment of the present disclosure, a liquid level detection (vLLD) function may be performed using the image acquisition unit 9 including a vision camera. According to an embodiment of the present disclosure, after centrifugation, a position of a cell pellet may be detected on the basis of an acquired image, and the remaining solution other than the cell pellet may be suctioned and then discharged. In other words, the centrifuged solution or the like may be photographed using the image acquisition unit 9, and the position of the cell pellet may be identified from the photographed image. Accordingly, a user may accurately extract an extraction target.

According to an embodiment of the present disclosure, the container having a predetermined shape may be a tubular container having a stopper, for example, an eppendorf tube (E-tube) or a centrifuge tube (C-tube). In S620, the material (e.g., a cell, a cell suspension, etc.) included in the 6-well plate cell container may be transferred to the container having a predetermined shape (e.g., a C-tube) positioned in the second work part 8.

When the robot arm 300 moves the 6-well plate cell container to the loader 5, the liquid handler 400 may move the cell container of the loader 5 to the first work part 7. In addition, the liquid handler 400 may move the C-tube of the C-tube storage 3 to the decapper 17 and may allow the decapper 17 to open the cap of the C-tube. The C-tube having the opened cap may be moved to the second work part 8 by the liquid handler 400. The cap of the 6-well plate cell container moved to the first work part 7 may be opened by the liquid handler 400. The first work part may be tilted, and the cell suspension in the tilted 6-well plate cell container may be transferred to the C-tube of the second work part 8 by the liquid handler 400. After the cell suspension is transferred, the cap of the 6-well plate cell container may be closed by the liquid handler 400.

The transferring of the material that is included in the container moved to the first work part 7 to the container having a predetermined shape positioned in the second work part 8 (S620) may further include dividing a certain solution into the container moved to the first work part 7, shaking the container to transfer a mixed liquid, and then transferring a mixed liquid to the container of a predetermined shape (e.g., the C-tube). And then, a supernatant liquid that is generated in the container through the centrifugation may be removed. After a predetermined material is injected into the C-tube and mixed, a user may wait for a predetermined time. The predetermined material may be an enzyme, etc., and the predetermined time is three minutes. However, the present disclosure is not limited thereto.

In addition, the liquid handler 400 may suction a phosphate-buffered saline (PBS) solution from the second culture medium (material) storage 12 and may divid the PBS solution into the 6-well plate cell container of the first work part 7. Since the first work part 7 may be tilted as described above, a shaking process may be performed by the first work part 7 moving while being repeatedly tilted. The liquid handler 400 may suction the PBS solution from the 6-well plate cell container of the first work part 7 and may divid the PBS solution into the C-tube provided in the second work part 8.

The processing of the particle separated through the centrifugation according to an embodiment of the present disclosure (S640) may include removing a supernatant liquid that is generated in the container through the centrifugation, injecting a predetermined material into the container having a predetermined shape to perform mixing and suspension, performing the centrifugation on the container that contains the suspended material, removing the supernatant liquid that is generated in the container through the centrifugation, injecting the culture medium into the container to perform mixing, and mixing the predetermined material with the cell suspension using another container having a predetermined shape.

In addition, the predetermined material may be an enzyme or phosphate-buffered saline (PBS).

According to an embodiment of the present disclosure, the suspension may be performed after a predetermined material (e.g., PBS) is added to the C-tube. In addition, the C-tube that contains the suspended material may be moved to the centrifugal separator 700, and the centrifugation may be performed on the C-tube. The mixing may be performed by removing the supernatant liquid that is generated in the container (e.g., C-tube) through the centrifugation and injecting the culture medium to the container.

As described above, the liquid handler 400 may move the C-tube having the supernatant liquid removed therefrom to the second work part 8. In addition, the liquid handler 400 may suction an enzyme from the second culture medium (material) storage 12, divid the enzyme into the C-tube of the second work part 8, and repeatedly perform suctioning and discharging from the C-tube to perform mixing. After the mixing, a user may wait for a predetermined time.

In addition, the liquid handler 400 may suction a PBS solution of the second culture medium (material) storage 12 and discharge the PBS solution into the C-tube provided in the second work part 8. The liquid handler 400 may repeatedly perform suction from and discharge to the C-tube provided in the second work part 8 to perform suspension. The C-tube accommodating the suspended material may be moved from the second work part 8 to the decapper 17 by the liquid handler 400, and the cap of the C-tube may be closed by the decapper 17.

The C-tube having the closed cap may be moved to the centrifugal separator 700 and then centrifuged. The supernatant liquid that is generated in the container (e.g., C-tube) through the centrifugation may be removed by the liquid handler 400.

The container (e.g., C-tube) that is centrifuged to have the supernatant liquid removed therefrom may be moved to the second work part 8 by the liquid handler 400. The liquid handler 400 may suction a culture medium from the second culture medium (material) storage 12 and divid the culture medium into the C-tube moved to the second work part 8. In addition, the liquid handler 400 may perform suction and discharging from the C-tube to perform mixing.

The liquid handler 400 may divid the PBS to the E-tube provided in the second work part 8. In addition, the liquid handler 400 may suction a cell suspension from the C-tube provided in the second work part 8 and divid the cell suspension into the E-tube. The mixing may be performed by repeating suction and discharging from the C-tube.

The observing of the processed particle using the microscope 200 according to an embodiment of the present disclosure may further include transferring the material included in the container having a predetermined shape to an auxiliary observation device before observing the processed particle through the microscope 200. The auxiliary observation device may include a microscopic chip (e.g., C-chip).

For example, the liquid handler 400 may suction trypan blue and divid the trypan blue to the auxiliary observation device (e.g., C-chip) positioned in the first work part 7. In addition, the liquid handler 400 may suction the cell suspension from the C-tube of the second work part 8, divid the cell suspension into the C-chip, and repeat suction and discharging from the C-chip to perform mixing. In addition, the liquid handler 400 may transfer a mixed liquid to another C-chip. Subsequently, the liquid handler 400 may move the C-chip to the loader 5. The C-chip moved to the loader 5 may be moved to the microscope 200 by the robot arm 300, and a state of the cell may be observed through the microscope 200. In other words, the control device 500 may perform cell counting by analyzing a microscopic image of a cell acquired through the microscope 200.

The transferring of the processed particle to a new container according to an embodiment of the present disclosure (S600) may further include dividing the culture medium into the new container before the transfer of the particle. The material including the processed particle may be transferred to the new container into which the culture medium is divided.

The robot arm 300 may move the new container (e.g., a 6-well plate) that is stored in the repository 600 to the loader 5. The new container moved to the loader 5 may be moved to the first work part 7 by the liquid handler 400. In addition, the cap of the new container may be opened. The liquid handler 400 may divid the culture medium of the second culture medium (material) storage 12 into the new container positioned in the first work part 7.

In addition, the liquid handler 400 may suction a cell suspension from the C-tube of the second work part 8 and divid the cell suspension into the new container of the first work part 7. Subsequently, the cap of the new container may be closed.

The liquid handler 400 may move the new container of the first work part 7 to the loader 5. The robot arm 300 may reinsert the new container of the loader 5 into the incubator 100.

A computer-readable medium may be any usable medium accessible by a computer and may include volatile and non-volatile media and discrete and integrated media. Also, the computer-readable medium may include both a computer storage medium and a communication medium. The computer storage medium includes the volatile and non-volatile media and the discrete and integrated media, which are implemented in any method or technique for storing information such as a computer readable instruction, data structure, program module, or other data. The communication module typically includes the computer readable instruction, data structure, program module, or other data and transmission mechanism of a modulated data signal such as a carrier and further includes any information transmission medium.

As described above, according to the one or more of the above exemplary embodiments, it is possible to systematically culture a cell by applying a certain culture protocol using the automatic cell culture device, thereby minimizing a deviation between experimenters.

It is also possible to safely culture a cell by minimizing experimental infection or tumor cell contact that may occur when a tumor cell is cultured.

In addition, by using the automatic cell culture device according to an embodiment of the present disclosure, it is possible to stably and precisely culture a cell, separate a cell (e.g., through the centrifugation, etc.), or process a cell (e.g., add an enzyme, perform pipetting, add a culture medium, etc.).

Furthermore, by using the automatic cell culture device according to an embodiment of the present disclosure, it is possible to quickly and efficiently dividing a trypan blue solution, which is used to check the ratio of dead cells to live cells, into cells and check the cells (e.g., counting, etc.).

## Claims

1. An automatic cell culture device comprising:
an incubator (100) configured to contain at least one container for culturing cells;
a microscope (200) configured to observe a state of the cell in the container;
a robot arm (300) configured to move the container;
a liquid handler (400) comprising a grip part (410), which is able to move forward or backward along a longitudinal axis road, and to move leftward or rightward along a transversal axis road, wherein the liquid handler (400) is configured to introduce liquid into or discharge liquid from the container and the grip part (410) is configured to hold and move the container;
a work part (30, 7) configured to perform at least one of rotating, shaking, and tilting of the container; and
a control device (500) configured to control an operation of at least one of the incubator (100), the microscope (200), the robot arm (300), the liquid handler (400), the grip part and the work part (30, 7).

2. The automatic cell culture device of claim 1, further comprising:
a repository (600) configured to store the container to be moved by the robot arm(300); and
a centrifugal separator (700) configured to separate a particle of a material included in the container using centrifugal force.

3. An operating method of an automatic cell culture device according to claim 1 or 2,
the automatic cell culture device comprising an incubator (100), a microscope (200), a robot arm (300), a liquid handler (400), a control device (500) and a centrifugal separator (700),
the operating method comprising:
extracting at least one container that contains a cell cultured in the incubator (100) for a predetermined time from the incubator(100) using the robot arm(300);
observing a state of the cell in the extracted container using the microscope (200);
selecting an operation protocol for the container based on a result of the observation;
and
driving the robot arm (300) according to the selected operation protocol,
wherein the grip part (410) transfers the container to the work part (30) of the automatic cell culture device configured to perform at least one of rotating, shaking, and tilting of the transferred container.

4. The operating method of claim 3, wherein the operation protocol for the container is at least one of a protocol for an operation of reinserting the container into the incubator (100), a protocol for an operation of injecting a culture medium into the container, and a protocol for an operation of performing sub-culturing using the container.

5. The operating method of claim 4, wherein,
when the protocol for an operation of injecting the cultural medium into the container is selected, the driving of the robot arm (300) comprises enabling the robot arm (300) to move the container extracted from the incubator (100) or the repository (600) to a loader (5), and
the operating method further comprises:
moving the container moved to the loader (5) to a first work part (7) using the grip part (410) included in the liquid handler (400);
suctioning a culture medium provided in a culture medium storage (10) using the liquid handler (400) and dividing the sucked culture medium into the container positioned in the first work part (7);
moving the container into which the culture medium is divided to the loader (5) using the grip part (410); and
reinserting the container moved to the loader (5) into the incubator (100).

6. The operating method of claim 4, wherein,
when the protocol for an operation of performing sub-culturing using the container is selected, the driving of the robot arm (300) comprises enabling the robot arm (300) to move the container extracted from the incubator (100) or the repository (600) to a loader (5), and
the operating method further comprises:
moving the container moved to the loader (5) to the first work part (7) using the grip part (410) included in the liquid handler (400);
transferring a material included in the container moved to the first work part (7) to a container having a predetermined shape positioned in a second work part (8);
moving the container having a predetermined shape to the centrifugal separator (700) to perform centrifugation on the container;
processing a particle separated through the centrifugation;
observing the processed particle using the microscope (200);
transferring the processed particle to a new container; and
reinserting the new container into the incubator (100).

7. The operating method of claim 6, wherein,
the container having a predetermined shape is a tubular container having a stopper, and the transferring of a material included in the container moved to the first work part (7) to a container having a predetermined shape positioned in the second work part (8) further comprises transferring a mixed liquid acquired by dividing a predetermined solution into the container moved to the first work part (7) and shaking the container to the container having a predetermined shape.

8. The operating method of claim 6, wherein the processing of a particle separated through the centrifugation comprises:
removing a supernatant liquid generated in the container through the centrifugation;
injecting a predetermined material into the container having a predetermined shape to perform mixing and suspension;
performing centrifugation on the container that contains the suspended material;
removing the supernatant liquid generated in the container through the centrifugation;
injecting the culture medium into the container to perform mixing; and
mixing the predetermined material and a cell suspension using another container having a predetermined shape.

9. The operating method of claim 8, wherein the predetermine material is an enzyme or a phosphate-buffered saline (PBS).

10. The operating method of claim 6, wherein,
the observing of the processed particle using the microscope (200) further comprises transferring the material included in the container having a predetermined shape to an auxiliary observation device before observing the processed particle through the microscope (200), and
the auxiliary observation device comprises a microscopic chip.

11. The operating method of claim 6, wherein,
the transferring of the processed particle to a new container further comprises dividing the culture medium into the new container before the transfer of the particle, and
the material including the processed particle is transferred to the new container into which the culture medium is divided.

12. A non-transitory computer-readable recording medium having recorded thereon a program for performing the method of any one of claims 3 to 11.

## Patentansprüche

1. Automatische Zellkulturvorrichtung, die Folgendes umfasst:
einen Inkubator (100), der dazu konfiguriert ist, mindestens einen Behälter für die Kultivierung von Zellen zu enthalten;
ein Mikroskop (200), das zur Beobachtung eines Zustands der Zelle in dem Behälter konfiguriert ist;
einen Roboterarm (300), der dazu konfiguriert ist, den Behälter zu bewegen;
eine Flüssigkeitshandhabungsvorrichtung (400), die ein Greifteil (410) umfasst, das in der Lage ist, sich entlang einer longitudinalen Achsenstraße vorwärts oder rückwärts zu bewegen und sich entlang einer transversalen Achsenstraße nach links oder rechts zu bewegen,
wobei die Flüssigkeitshandhabungsvorrichtung (400) dazu konfiguriert ist, Flüssigkeit in den Behälter einzubringen oder aus ihm abzuführen, und das Greifteil (410) dazu konfiguriert ist, den Behälter zu halten und zu bewegen;
ein Arbeitsteil (30, 7), das dazu konfiguriert ist, ein Drehen, Schütteln und/oder Kippen des Behälters durchzuführen; und
eine Steuer/Regelvorrichtung (500), die dazu konfiguriert ist, einen Betrieb des Inkubators (100), des Mikroskops (200), des Roboterarms (300), der Flüssigkeitshandhabungsvorrichtung (400), des Griffteils und des Arbeitsteils (30, 7) zu steuern/regeln.

2. Automatische Zellkulturvorrichtung nach Anspruch 1, ferner umfassend
ein Lager (600), das so konfiguriert ist, dass es den durch den Roboterarm (300) zu bewegenden Behälter lagert; und
einen Zentrifugalseparator (700), der so konfiguriert ist, dass er ein Partikel eines in dem Behälter enthaltenen Materials unter Verwendung von Zentrifugalkraft abtrennt.

3. Betriebsverfahren für eine Automatische Zellkulturvorrichtung nach Anspruch 1 oder 2, wobei die automatische Zellkulturvorrichtung einen Inkubator (100), ein Mikroskop (200), einen Roboterarm (300), eine Flüssigkeitshandhabungsvorrichtung (400), eine Steuer/Regelvorrichtung (500) und einen Zentrifugalseparator (700) umfasst,
wobei das Betriebsverfahren Folgendes umfasst:
Entnehmen mindestens eines Behälters, der eine in dem Inkubator (100) für eine vorbestimmte Zeit kultivierte Zelle enthält, aus dem Inkubator (100) unter Verwendung des Roboterarms (300);
Beobachten eines Zustands der Zelle in dem entnommenen Behälter unter Verwendung des Mikroskops (200);
Auswählen eines Betriebsprotokolls für den Behälter basierend auf einem Ergebnis der Beobachtung; und
Antreiben des Roboterarms (300) gemäß dem ausgewählten Betriebsprotokoll, wobei das Greifteil (410) den Behälter zu dem Arbeitsteil (30) der automatischen Zellkulturvorrichtung überführt, der dazu konfiguriert ist, ein Drehen, Schütteln und/oder Kippen des überführten Behälters durchzuführen.

4. Betriebsverfahren nach Anspruch 3, wobei das Betriebsprotokoll für den Behälter ein Protokoll für einen Vorgang des Wiedereinsetzens des Behälters in den Inkubator (100), ein Protokoll für einen Vorgang des Injizierens eines Kulturmediums in den Behälter und/oder ein Protokoll für einen Vorgang des Durchführens einer Sub-Kultur unter Verwendung des Behälters ist.

5. Betriebsverfahren nach Anspruch 4, wobei,
wenn das Protokoll für einen Vorgang des Injizierens des Kulturmediums in den Behälter ausgewählt wird, das Antreiben des Roboterarms (300) es umfasst, den Roboterarm (300) zu befähigen, den aus dem Inkubator (100) oder dem Lager (600) entnommenen Behälter zu einem Belader (5) zu bewegen, und
das Betriebsverfahren ferner umfasst:
Bewegen des zum Belader (5) bewegten Behälters zu einem ersten Arbeitsteil (7) unter Verwendung des in der Flüssigkeitshandhabungsvorrichtung (400) enthaltenen Greifteils (410);
Ansaugen eines in einem Kulturmedium-Speicher (10) bereitgestellten Kulturmediums unter Verwendung der Flüssigkeitshandhabungsvorrichtung (400) und Aufteilen des angesaugten Kulturmediums in den im ersten Arbeitsteil (7) positionierten Behälter;
Bewegen des Behälters, in den das Kulturmedium aufgeteilt ist, zu dem Belader (5) unter Verwendung des Greifteils (410); und
Wiedereinsetzen des zum Belader (5) bewegten Behälters in den Inkubator (100).

6. Betriebsverfahren nach Anspruch 4, wobei
wenn das Protokoll für einen Vorgang des Durchführens einer Subkultivierung unter Verwendung des Behälters ausgewählt wird, das Antreiben des Roboterarms (300) es umfasst, den Roboterarm (300) zu befähigen, den aus dem Inkubator (100) oder dem Lager (600) entnommenen Behälter zu einem Belader (5) zu bewegen, und
das Betriebsverfahren ferner umfasst:
Bewegen des zum Belader (5) bewegten Behälters zum ersten Arbeitsteil (7) unter Verwendung des in der Flüssigkeitshandhabungsvorrichtung (400) enthaltenen Greifteils (410);
Überführen eines Materials, das in dem zu dem ersten Arbeitsteil (7) bewegten Behälter enthalten ist, in einen Behälter mit einer vorbestimmten Form, der in einem zweiten Arbeitsteil (8) positioniert ist;
Bewegen des Behälters mit einer vorbestimmten Form zu dem Zentrifugalseparator (700), um eine Zentrifugation an dem Behälter durchzuführen;
Verarbeiten eines durch die Zentrifugation abgetrennten Partikels;
Beobachten des verarbeiteten Partikels unter Verwendung des Mikroskops (200);
Überführen des verarbeiteten Partikels in einen neuen Behälter; und
Wiedereinsetzen des neuen Behälters in den Inkubator (100).

7. Betriebsverfahren nach Anspruch 6, wobei,
der Behälter, der eine vorbestimmte Form aufweist, ein rohrförmiger Behälter mit einem Stopfen ist, und das Überführen eines in dem zum ersten Arbeitsteil (7) bewegten Behälter enthalten Materials in einen Behälter mit einer vorbestimmten Form, der im zweiten Arbeitsteil (8) positioniert ist, ferner das Überführen einer gemischten Flüssigkeit, die durch Aufteilen einer vorbestimmten Lösung gewonnen wurde, in den zum ersten Arbeitsteil (7) bewegten Behälter und Schütteln des Behälters in den Behälter mit einer vorbestimmten Form umfasst.

8. Betriebsverfahren nach Anspruch 6, wobei das Verarbeiten eines durch die Zentrifugation abgetrennten Partikels umfasst:
Entfernen eines im Behälter durch die Zentrifugation erzeugten Flüssigkeitsüberstands;
Injizieren eines vorbestimmten Materials in den Behälter, der eine vorbestimmte Form aufweist, um ein Mischen und Suspendieren durchzuführen;
Durchführen einer Zentrifugation an dem Behälter, der das suspendierte Material enthält;
Entfernen des in dem Behälter durch die Zentrifugation erzeugten Flüssigkeitsüberstands;
Injizieren des Kulturmediums in den Behälter, um ein Mischen durchzuführen; und
Mischen des vorbestimmten Materials und einer Zellsuspension unter Verwendung eines weiteren Behälters mit einer vorbestimmten Form.

9. Betriebsverfahren nach Anspruch 8, wobei das vorbestimmte Material ein Enzym oder eine phosphatgepufferte Salzlösung (PBS) ist.

10. Betriebsverfahren nach Anspruch 6, wobei,
das Beobachten des verarbeiteten Partikels unter Verwendung des Mikroskops (200) ferner das Überführen des in dem Behälter mit einer vorbestimmten Form enthaltenen Materials zu einer Hilfsbeobachtungsvorrichtung vor dem Beobachten des verarbeiteten Partikels durch das Mikroskop (200) umfasst, und
die Hilfsbeobachtungsvorrichtung einen mikroskopischen Chip umfasst.

11. Betriebsverfahren nach Anspruch 6, wobei
das Überführen des verarbeiteten Partikels in einen neuen Behälter ferner das Aufteilen des Kulturmediums in den neuen Behälter vor dem Überführen des Partikels umfasst, und
das Material, das das verarbeitete Partikel enthält, in den neuen Behälter überführt wird, in den das Kulturmedium aufgeteilt wird.

12. Nicht-transitorisches computerlesbares Aufzeichnungsmedium, auf dem ein Programm zur Durchführung des Verfahrens nach einem der Ansprüche 3 bis 11 aufgezeichnet ist.

## Revendications

1. Dispositif de culture cellulaire automatique comprenant :
un incubateur (100) configuré pour contenir au moins un contenant pour la mise en culture de cellules ;
un microscope (200) configuré pour observer un état de la cellule dans le contenant ;
un bras de robot (300) configuré pour déplacer le contenant ;
un manipulateur de liquide (400) comprenant une partie de préhension (410), qui est apte à se déplacer vers l'avant ou vers l'arrière le long d'un chemin à axe longitudinal, et à se déplacer vers la gauche ou vers la droite le long d'un chemin à axe transversal, dans lequel le manipulateur de liquide (400) est configuré pour introduire du liquide dans le contenant ou évacuer du liquide à partir de celui-ci et la partie de préhension (410) est configurée pour tenir et déplacer le contenant ;
une partie de travail (30, 7) configurée pour réaliser au moins une parmi la rotation, l'agitation et l'inclinaison du contenant ; et
un dispositif de commande (500) configuré pour commander une opération d'au moins un parmi l'incubateur (100), le microscope (200), le bras de robot (300), le manipulateur de liquide (400), la partie de préhension et la partie de travail (30, 7).

2. Dispositif de culture cellulaire automatique selon la revendication 1, comprenant en outre :
un dépôt (600) configuré pour stocker le contenant devant être déplacé par le bras de robot (300) ; et
un séparateur centrifuge (700) configuré pour séparer une particule d'un matériau inclus dans le contenant à l'aide d'une force centrifuge.

3. Procédé de fonctionnement d'un dispositif de culture cellulaire automatique selon la revendication 1 ou 2,
le dispositif de culture cellulaire automatique comprenant un incubateur (100), un microscope (200), un bras de robot (300), un manipulateur de liquide (400), un dispositif de commande (500) et un séparateur centrifuge (700),
le procédé de fonctionnement comprenant :
l'extraction d'au moins un contenant, qui contient une cellule mise en culture dans l'incubateur (100) pendant une durée prédéterminée, à partir de l'incubateur (100) à l'aide du bras de robot (300) ;
l'observation d'un état de la cellule dans le contenant extrait à l'aide du microscope (200) ;
la sélection d'un protocole d'opération pour le contenant sur la base d'un résultat de l'observation ;
et
l'entraînement du bras de robot (300) selon le protocole d'opération sélectionné,
dans lequel la partie de préhension (410) transfère le contenant vers la partie de travail (30) du dispositif de culture cellulaire automatique configurée pour réaliser au moins une parmi la rotation, l'agitation et l'inclinaison du contenant transféré.

4. Procédé de fonctionnement selon la revendication 3, dans lequel le protocole d'opération pour le contenant est au moins un parmi un protocole pour une opération de réinsertion du contenant dans l'incubateur (100), un protocole pour une opération d'injection d'un milieu de culture dans le contenant, et un protocole pour une opération de mise en sous-culture à l'aide du contenant.

5. Procédé de fonctionnement selon la revendication 4, dans lequel,
lorsque le protocole pour une opération d'injection du milieu de culture dans le contenant est sélectionné, l'entraînement du bras de robot (300) comprend le fait de permettre au bras de robot (300) de déplacer le contenant extrait à partir de l'incubateur (100) ou du dépôt (600) vers un chargeur (5), et
le procédé de fonctionnement comprend en outre :
le déplacement du contenant déplacé vers le chargeur (5) vers une première partie de travail (7) à l'aide de la partie de préhension (410) incluse dans le manipulateur de liquide (400) ;
l'aspiration d'un milieu de culture ménagé dans un stockage de milieu de culture (10) à l'aide du manipulateur de liquide (400) et la division du milieu de culture aspiré dans le contenant positionné dans la première partie de travail (7) ;
le déplacement du contenant dans lequel le milieu de culture est divisé vers le chargeur (5) à l'aide de la partie de préhension (410) ; et
la réinsertion du contenant déplacé vers le chargeur (5) dans l'incubateur (100).

6. Procédé de fonctionnement selon la revendication 4, dans lequel,
lorsque le protocole pour une opération de mise en sous-culture à l'aide du contenant est sélectionné, l'entraînement du bras de robot (300) comprend le fait de permettre au bras de robot (300) de déplacer le contenant extrait à partir de l'incubateur (100) ou du dépôt (600) vers un chargeur (5), et
le procédé de fonctionnement comprend en outre :
le déplacement du contenant déplacé vers le chargeur (5) vers la première partie de travail (7) à l'aide de la partie de préhension (410) incluse dans le manipulateur de liquide (400) ;
le transfert d'un matériau inclus dans le contenant déplacé vers la première partie de travail (7) vers un contenant ayant une forme prédéterminée positionné dans une seconde partie de travail (8) ;
le déplacement du contenant ayant une forme prédéterminée vers le séparateur centrifuge (700) pour réaliser une centrifugation sur le contenant ;
le traitement d'une particule séparée par le biais de la centrifugation ;
l'observation de la particule traitée à l'aide du microscope (200) ;
le transfert de la particule traitée vers un nouveau contenant ; et
la réinsertion du nouveau contenant dans l'incubateur (100).

7. Procédé de fonctionnement selon la revendication 6, dans lequel,
le contenant ayant une forme prédéterminée est un contenant tubulaire ayant un bouchon, et le transfert d'un matériau inclus dans le contenant déplacé vers la première partie de travail (7) vers un contenant ayant une forme prédéterminée positionné dans la seconde partie de travail (8) comprend en outre le transfert d'un liquide mélangé acquis par la division d'une solution prédéterminée dans le contenant déplacé vers la première partie de travail (7) et l'agitation du contenant vers le contenant ayant une forme prédéterminée.

8. Procédé de fonctionnement selon la revendication 6, dans lequel le traitement d'une particule séparée par le biais de la centrifugation comprend :
le retrait d'un liquide surnageant généré dans le contenant par le biais de la centrifugation ;
l'injection d'un matériau prédéterminé dans le contenant ayant une forme prédéterminée pour réaliser un mélange et une mise en suspension ;
la réalisation d'une centrifugation sur le contenant qui contient le matériau en suspension ;
le retrait du liquide surnageant généré dans le contenant par le biais de la centrifugation ;
l'injection du milieu de culture dans le contenant pour réaliser un mélange ; et
le mélange du matériau prédéterminé et d'une suspension cellulaire à l'aide d'un autre contenant ayant une forme prédéterminée.

9. Procédé de fonctionnement selon la revendication 8, dans lequel le matériau prédéterminé est une enzyme ou une solution saline à tampon phosphate (PBS).

10. Procédé de fonctionnement selon la revendication 6, dans lequel,
l'observation de la particule traitée à l'aide du microscope (200) comprend en outre le transfert du matériau inclus dans le contenant ayant une forme prédéterminée vers un dispositif d'observation auxiliaire avant l'observation de la particule traitée par le biais du microscope (200), et
le dispositif d'observation auxiliaire comprend une puce microscopique.

11. Procédé de fonctionnement selon la revendication 6, dans lequel,
le transfert de la particule traitée vers un nouveau contenant comprend en outre la division du milieu de culture dans le nouveau contenant avant le transfert de la particule, et
le matériau incluant la particule traitée est transféré vers le nouveau contenant dans lequel le milieu de culture est divisé.

12. Support d'enregistrement non transitoire lisible par ordinateur sur lequel est enregistré un programme pour réaliser le procédé selon l'une quelconque des revendications 3 à 11.
